# EUROPEAN PATENT APPLICATION

(11) **EP 1 632 503 A1**
(43) Date of publication of application: **08.03.2006**
(21) Application number: 04425460.5
(22) Date of filing: 25.06.2004
(51) Int. Cl.: C08B 37/16, B01J 20/292, C08G 64/02, A61K 47/48, B01J 20/26

(54) **Ultrasound-assisted synthesis of cyclodextrin-based nanosponges**

(71) Applicant: SEA MARCONI TECHNOLOGIES DI WANDER TUMIATTI S.A.S., 10141 Torino (IT)
(72) Inventor: Trotta, Francesco, 10141 Torino (IT); Cavalli, Roberta, Via Pietro Giuria, 9 10125 Torino (IT); Tumiatti, Wander, 10141 Torino (IT); Zerbinati, Orfeo, 10141 Torino (IT); Roggero, Carlo, 10141 Torino (IT); Vallero, Roberto, 10141 Torino (IT)
(74) Representative: Minoja, Fabrizio

(57) **Abstract**

A description is given of substantially spherical nanosponges which can be obtained by crosslinking cyclodextrins and their by-products with organic carbonates as crosslinkers and ultrasounds without a solvent.

## Description

The present invention relates to nanosponges based on cyclodextrins (CD) which can be obtained through a reaction with organic carbonates, and their use as agents able to carry active ingredients or as decontaminants.

The use of nanosponges based on cyclodextrins obtained using organic carbonates as crosslinkers was initially aimed with success at eliminating the chlorinated aromatic compounds present in traces in water and in general at eliminating chlorinated hydrophobic organic molecules not present in water, but also in the soil and air, as contaminants at levels of concentration of the order of ppb-ppm.

It has now been discovered that nanosponges, above all in the form of microspheres, obtained with the present invention, are also able to carry active ingredients.

The active ingredients administered are distributed in the organism above all according to their chemical and physical features and their molecular structure. In most cases this distribution is not optimal and the quantity of drug which reaches the site of action is only a small fraction of the dose administered. For this reason various therapy systems have been developed, such as liposomes, micelles, micro and nanoparticles of a polymeric or lipidic nature for the carrying of drugs. The main purpose of these systems is that of optimising the chemical and physical and biopharmaceutical properties (such as bioavailability, administration route and dosage) of the active ingredients.

Oral administration represents the easiest and most convenient route for access to systemic circulation but may have some disadvantages, alongside problems linked to bioavailability, such as for example degradation by enzymes or the gastroenteric pH of the active ingredient administered. Following oral administration, the processes which affect absorption of a drug include not only its release from the pharmaceutical form, generally due to phenomena of dissolving, and its capacity for permeation through the intestinal barrier, but also its stability in the gastroenteric tract. The portion of drug able to arrive in an active form in the systemic circulation will give rise to the biological availability or bioavailability of the drug. In order to be able to be absorbed and have a therapeutic effect, the active ingredient must be dispersed at molecular level or completely solubilised in the body liquids. Consequently drugs with low solubility are formulated and administered at higher doses than those really necessary for performing the therapeutic action. This may lead to problems of toxicity and of patient compliance.

On these bases, one of the main problems to be solved in the pharmaceutical field is the increase in the solubility of active ingredients with low solubility in aqueous fluids such as physiological liquids.

The solubility of a substance is the factor limiting its application in therapy even when it has interesting pharmacodynamic properties. Therefore increasingly new strategies are being investigated to improve the solubility and kinetics of release of active ingredients.

To improve solubility in the technological pharmaceutical field various formulation approaches are adopted, such as the use of cosolvents, surfactants, particulate systems and complexes. Inclusion complexes - active ingredients and cyclodextrins - represent one of the most interesting approaches.

Cyclodextrins (CD) are non-reducing cyclic oligosaccharides formed by 6-8 glucose molecules bonded with a 1,4-α-glucosidicic bond, having a characteristic truncated cone structure. The arrangement of the functional groups of the glucose molecules is such that the surface of the molecule is polar while the internal cavity is lipophilic.

The lipophilic cavity provides the CDs with the ability to form inclusion complexes which are also stable in solution with organic molecules of suitable polarity and size.

For this reason the CDs have already been studied and have numerous applications in various fields of chemistry (pharmaceutical, analytical, catalysis, cosmetic etc.) wherein the properties of the inclusion compounds are exploited.

In pharmaceutical technology these complexes can be adopted for increasing the speed of dissolving, solubility and stability of drugs, or for masking unpleasant tastes or for converting liquid substances into solids.

Their use has recently also been extended to decontamination from organic pollutants (e.g. POPs, PCBs) after the polymerisation required for increasing the value of the inclusion constant and making them insoluble in water. The polymerisation was achieved by using diisocyanates, epichlorohydrin or organic carbonates. In no case is their use as carriers of drugs and/or of active ingredients reported.

By covalently binding several molecules of functionalised cyclodextrin systems can be obtained, known as nanosponges, which can have suitable characteristics for use as carriers of active ingredients.

Given their characteristics the nanosponges could be applied to solving the intrinsic problems of the active ingredient such as the poor hydrosolubility, instability, degradation, protection and toxicity.

They could also carry simultaneously both lipophilic molecules in the hydrophobic cavity of the cyclodextrin and hydrophilic molecules in the spaces between the single cyclodextrins.

Nanosponges have colloidal dimensions and form clear and opalescent suspensions in water.

Following the process of synthesis developed for their preparation, the nanosponges can solidify into particles whose morphology is found to be spherical following observations at the optical microscope.

This characteristic of the synthesised nanosponges, together with suitable values of density, could be interesting for their possible application also as carriers via the inhalation route in addition to oral administration.

The object of the invention is nanosponges based on cyclodextrins obtained using organic carbonates as crosslinkers, operating in particular with the aid of ultrasounds without a solvent.

The synthesis of nanosponges using organic carbonates in solution thermally is a known process which involves the use of solvents such as DMF or DMSO in which both reagents are soluble. The reaction is carried out at temperatures higher than 130-140°C and leads to the formation of a reticulate which has proved to be active in the complexing of numerous organic molecules.

The product which is the object of the invention was obtained by a reaction between the cyclodextrin (CD) and the diphenyl carbonate (DPC) or other organic carbonates without a solvent operating at various temperatures between the ambient temperature and 90°C under sonication.

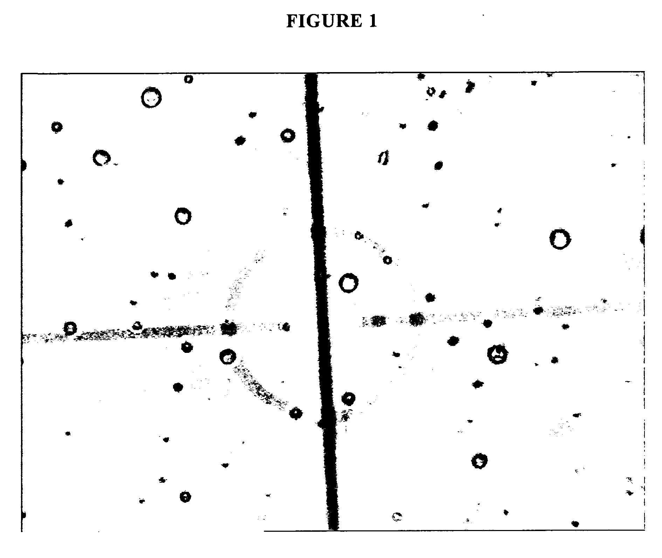

The product, which has suitable characteristics for its use as carrier of active ingredients, shows under the optical microscope a peculiar morphology: it is made up of spheroidal particles with regular dimensions smaller than 5 microns, as shown by way of an example in Figure 1.

The spherical shape of the individual particles is an essential condition for the polymer to be used for very advanced and innovative pharmaceutical applications in pharmaceutical technology.

The nanosponges produced with the assistance of ultrasounds according to the present invention are able to bond numerous organic compounds such as PCB, chlorinated and aromatic organic solvents, phthalates, POPs and PAH (aromatic hydrocarbons) and can therefore be used as decontaminating agents, similarly to what is disclosed in US 5425881, WO 03/085002 and DE 10008508.

The examples given below fully illustrate the features of the invention.

### Example 1

4.54 g (0.001 mols) of anhydrous β-CD and 0.856 g (0.004 mols) of diphenyl carbonate are mixed in a 250 ml flask. The flask is placed in an ultrasound bath filled with water and heated to 90°C. The mixture is sonicated for 5 h.

The reaction mixture is left to cool and the product obtained is broken up roughly. Numerous needle-shaped crystals of phenol can be seen on the clear surface and the neck of the flask and part of the phenol developed contributes to agglomerating the product.

The product is washed with water in order to remove the non-reacted cyclodextrin and then is washed in Soxhlet with ethanol to remove the phenol developed and the residual DPC.

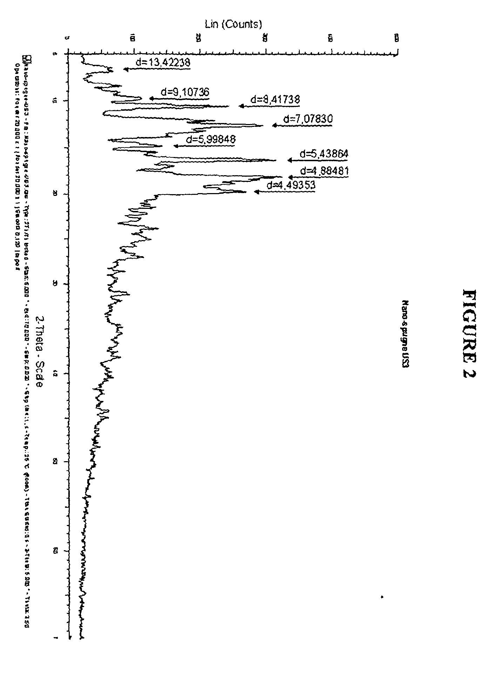

The product obtained is a fine white powder insoluble in water. Observations at the optical microscope (Figure 1) show the perfect spherical shape of the particles and their average diameter smaller than 5 microns and the low rate of polydispersity. Moreover the microspherical nanosponges have a high degree of crystallinity at low values of θ as can be seen from the X-ray analysis of the sample as in Example 3 (Figure 2).

### Example 2

100 ml of DMF, 4.54 g (0.001 mols) of anhydrous β-CD and 0.856 g (0.004 mols) of diphenyl carbonate are placed in a 250 ml flask. The flask is placed in an ultrasound bath filled with water and heated to 90°C. The mixture is sonicated for 5 h.

The reaction mixture is allowed to cool and is concentrated to a small volume in the rotavapor. At the end excess water is added, with filtering and washing with water for a long period and the product obtained is dried.

The product obtained is a fine white powder insoluble in water.

### Example 3

4.54 g (0.001 mols) of anyhdrous β-CD and 0.428 g (0.002 mols) of diphenyl carbonate are mixed in a 250 ml flask. The flask is placed in an ultrasound bath filled with water and heated to 90°C. The mixture is sonicated for 5 h.

The reaction mixture is left to cool and the product obtained is broken up roughly. Numerous needle-shaped crystals of phenol can be seen on the clear surface and the neck of the flask and part of the phenol developed contributes to agglomerating the product.

The product is washed with water in order to remove the non-reacted cyclodextrin then is cleansed of the phenol developed by evaporation in a nitrogen flow at 130°C.

The product obtained is a fine white powder not soluble in water and common organic solvents.

### Example 4

20 g (0.0176 mols) of anhydrous β-CD and 7.54 g (0.0352 mols) of diphenyl carbonate are mixed in a 100 ml beaker. The beaker is placed in an oil bath bain-marie and heated to 90°C. The mixture is sonicated for 4 h at 19kHz using an ultrasound probe capable of supplying a maximum power of 250W at 19kHz.

The reaction mixture is left to cool and the product obtained is broken up roughly. Numerous needle-shaped crystals of phenol can be seen on the clear surface of the beaker and part of the phenol developed contributes to agglomerating the product.

The product is washed with water in order to remove the non-reacted cyclodextrin and then is washed in Soxhlet with acetone to remove the phenol developed and the residual DPC.

The product obtained is a fine white powder not soluble in water and common organic solvents.

### Example 5

20 g (0.0176 mols) of anhydrousβ-CD and 11.30 g (0.0528 mols) of diphenyl carbonate are mixed in a 100 ml beaker. The beaker is placed in an oil bath bain-marie and heated to 90°C. The mixture is sonicated for 4 h at 19kHz using an ultrasound probe capable of supplying a maximum power of 250W at 19kHz.

The reaction mixture is left to cool and the product obtained is broken up roughly. Numerous needle-shaped crystals of phenol can be seen on the clear surface of the beaker and part of the phenol developed contributes to agglomerating the product.

The product is washed with water in order to remove the non-reacted cyclodextrin and then is washed in Soxhlet with acetone to remove the phenol developed and the residual DPC.

The product obtained is a fine white powder not soluble in water and common organic solvents.

### Example 6

10 g (0.0088 mols) of anhydrous β-CD and 9.416 g (0.044 mols) of diphenyl carbonate are mixed in a 100 ml beaker. The mixture is sonicated for 5 h at 19kHz at ambient temperature using an ultrasound probe capable of supplying a maximum power of 250W at 19kHz.

The reaction mixture is left to cool and the product obtained is broken up roughly. Numerous needle-shaped crystals of phenol can be seen on the clear surface of the beaker and part of the phenol developed contributes to agglomerating the product.

The product is washed with water in order to remove the non-reacted cyclodextrin and then is washed in Soxhlet with acetone to remove the phenol developed and the residual DPC.

The product obtained is a fine white powder not soluble in water and common organic solvents.

### Example 7

0.25 g of polymer are added to 10 ml of water contaminated with 19 ppm of chlorobenzene, as in example 1. Various samples are taken in time and each sample was analysed by UV-vis spectroscopy. The concentration of chlorobenzene was found to be 8.2 ppm after 30 minutes and 3.1 ppm after 180 minutes.

Abatement after 3 hours was found to be 84% approximately.

### Example 8

2 g of polymer are added to 50 ml of water contaminated with a mixture of chlorobenzene (820 ppb), 4-chlorotoluene (830 ppb), 2,6-dichlorotoluene (770 ppb), 1,3,5-trichlorobenzene (540 ppb) and hexachlorobenzene (225 ppb), as in example 3. Several samples are taken in time and each sample is analysed by gas chromatography-mass spectrometry. The total concentration of organics after 50 minutes is 180 ppb with an abatement of 94% approximately.

Considering the individual constituents a high efficiency is obtained for the more chlorinated compounds for which abatement higher than 97% is obtained.

### Example 9

2 g of polymer are added to 50 ml of water contaminated with 316 ppb of PCB (commercial mixture Aroclor 1242), as in example 4.

Several samples are taken in time and each sample is analysed by gas chromatography-mass spectrometry. The total concentration of PCB after 50 minutes was 32 ppb with an abatement of 90% approximately.

### Example 10

100 mg of nanosponges are added to 3 ml of water containing 20 mg of Flurbiprofen, as in example 3. This is left to be magnetically stirred for a night at ambient temperature. At the end it is filtered and the solid phase recovered and lyophilised. The nanosponges incorporate 10 mg of Flurbiprofen/100 mg.

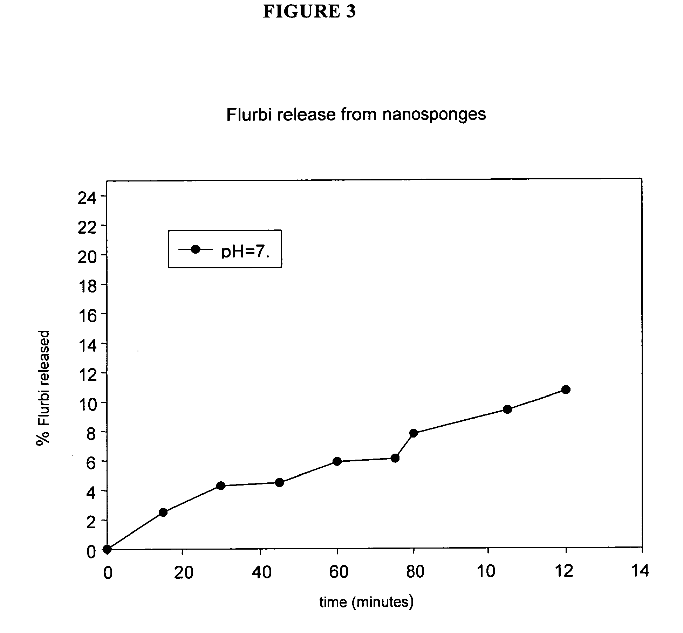

The lyophilised substance is subjected to release tests in a pH 7.4 buffer, while stirring and at a temperature of 3°C. Figure 3 gives the results obtained where the progressive and constant release of the active ingredient considered can be seen clearly.

### Example 11

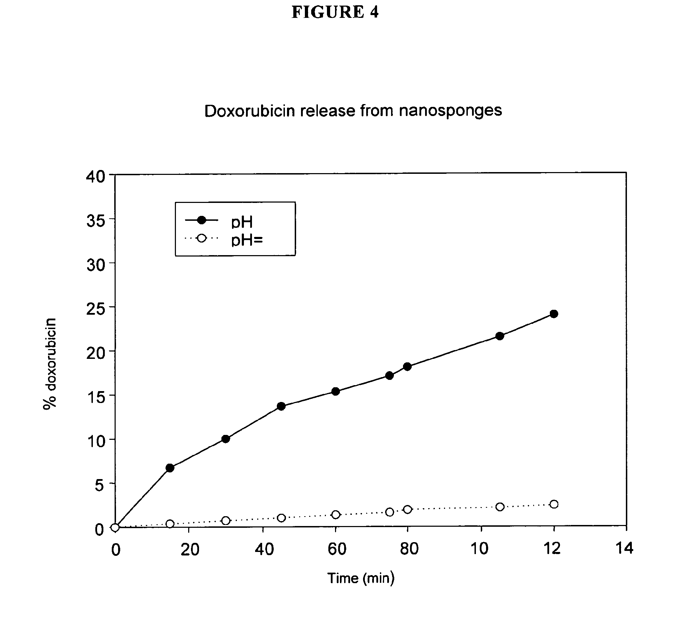

50 mg of nanosponges are added to 3 ml of water containing 3 mg of Doxorubicin, as in example 3. This is left to be magnetically stirred for a night at ambient temperature. At the end it is filtered and the solid phase recovered and lyophilised. The lyophilised substance incorporates 1% in weight of Doxorubicin. The lyophilised substance is subjected to tests of release in two different aqueous buffers. Figure 4 gives the results obtained where the progressive and constant release of the active ingredient considered can be seen clearly. It should be noted that the release depends largely on the pH used. With decidedly acid pH (gastric environment) the kinetics of release is slow. At pH 7.4 the release is considerable with faster release kinetics. This experiment proves that the nanosponges are able to carry active ingredients, passing unchanged through the gastric environment of the human stomach and releasing the active ingredients in the intestine. The nanosponges are therefore configured as carriers of active ingredients. The results of controlled and prolonged release in time are of particular importance considering the poor haemolytic activity of the nanosponges used as described in greater detail in example 12.

### Example 12

To determine haemolytic activity 250 µl of blood, obtained from donors, are added in a set of test tubes to increasing quantities of NANO-CD and then brought to 1 ml with PBS 10 mM pH=7.4 sterile buffer. The quantities of NANO-CD considered are: 0.5 mg, 1.0 mg, 1.5 mg, 2.0 mg, 2.5 mg, 5.0 mg, 7.5 mg, 10.0 mg, 15.0 mg, 20.0 mg and 25.0 mg. To this set of test tubes 1 test tube is added containing 250 µl of blood and 750 µl of PBS 10 mM pH=7.4 sterile buffer used as a control and a test tube containing 250 µl of blood and 750 µl of PBS 10 mM pH=7.4 sterile buffer to which excess ammonium chloride is added to obtain total haemolysis of the red globules present.

The test tubes are then incubated for 90 minutes at a temperature of 37°C. After 90 minutes centrifuging is carried out at 2000 rpm for 10 minutes; 250 µl of the surnatant are taken and placed in a quartz cuvette containing 2.5 ml of a 10 mM pH 7.4 sterile phosphate buffer. As a reference the sample of blood is used and in which total haemolysis is caused by adding ammonium chloride. All the samples are then analysed at the spectrophotometer (Lambda 2, Perkin Elmer) at the wavelength of 543 nm. The percentage of haemolysis is calculated from the absorbance due to the presence of haemoglobin in the surnatant. 100% haemolysis corresponds to 543 nm absorbance of the fully haemolysed sample (reference).

No haemolytic activity occurs up to the concentration of 6 mg/ml.

## Claims

1. Nanosponges of a substantially spherical shape which can be obtained through crosslinking of cyclodextrins and their by-products with organic carbonates as crosslinkers and ulstrasounds without a solvent.

2. Nanosponges according to claim 1 of a spheroidal shape and dimensions smaller than 5 microns.

3. Nanosponges according to claim 1 or 2 wherein the cyclodextrin is a beta-cyclodextrin and the carbonate is diphenyl carbonate.

4. Use of the nanosponges of claim 1, 2 or 3 as carriers of active ingredients of pharmaceutical, food, cosmetic and agrarian interest (phytopharmaceuticals).

5. Use of the nanosponges of claim 1 or 2 as decontaminant agents in respect of polychlorobiphenyls (PCB), chlorinated and aromatic organic solvents, phthalates, persistent organic pollutants (POPs), PAH and inorganic types.

6. Compositions comprising active ingredients included in the nanosponges of claims 1-3.

7. Nanosponges according to claim 1 or 2 for use in analytical chemistry as stationary phases for chromatography.

8. Nanosponges according to claim 1 or 2 as excipients for preparing tablets, pellets, granules with sizes between 0.5 mm and 20 mm and powder, also for inhalation administration.

9. Nanosponges according to claim 1 or 2 for extractions of vegetable and/or animal active ingredients.

10. Nanosponges according to claim 1 or 2, magnetised for complexing of active ingredients and their carrying and application.

11. Nanosponges according to claim 1 or 2 for the treatment of environmental matrices such as air, water, soil and surfaces.

12. Nanosponges according to claim 1 or 2 for the removal of organic and inorganic radioactive substances and in particular radioactive elementary iodine.

13. Regeneration of nanosponges by simple thermal desorption, extraction with solvents and/or use of microwaves and ultrasounds.

14. Nanosponges according to claim 1 or 2 for use in individual systems of protection against chemical attack and agents.

15. Nanosponges according to claim 1 or 2 for the purification of water.

16. Nanosponges according to claim 1 or 2 for use in the processes of deodorising liquid and/or gaseous effluent.
